# EUROPEAN PATENT APPLICATION

(11) **EP 1 986 115 A1**
(43) Date of publication of application: **29.10.2008**
(21) Application number: 07105665.9
(22) Date of filing: 04.04.2007
(51) Int. Cl.: G06F 19/00

(54) **Determination of group-specific biomarkers**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention discloses a (computer) model that describes molecular biochemical processes in the body that are relevant for a specific disease and can be based on a combination of a priori knowledge (e.g. from literature) and specific clinical data (e.g. from a disease focused clinical studies). The invention aims to find biomarkers that are relevant for a specific disease by creating different models for different patient groups or categories, e.g. relating to "healthy" and "affected" or to different stages of a disease. Each model is based on the same underlying a priori knowledge, but on different pools of group-specific clinical data. Biomarkers are identified through model comparison, with a special interest for local differences (i.e. in a specific, limited part of the model).

## Description

The invention relates to a method for the determination of biomarkers for different patient groups with respect to a given specific disease.

Modem day diagnostics can draw from a large variety of patient data, including high throughput molecular data, imaging data and huge amounts of background knowledge. Since knowledge is not necessarily wisdom, a resulting challenge is to translate this avalanche of available data into small pieces of relevant information. In the field of diagnostics, such pieces of information may take the form of biomarkers, i.e. a selection and/or transformation of the full offer of available information (e.g. micro-array or proteomics data) that can be used to make clear statements about a patient. Such statements may include a classification of the patient (e.g. into "healthy", "diseased", "severely afflicted" or "mildly afflicted") or the indication of treatment success or failure.

Current practice in biomarker discovery is to perform the same series of measurements (e.g. using the same micro-array setup) for two different groups of patients (e.g. affected and non-affected individuals), and then to check each feature in the measurement (e.g. the expression for each single gene) for relevant differences between the two groups of patients. Because of the generally large number of such features (about 25000 for a full genome micro-array) and the often low number of study subjects/patients (generally order 100) it is very hard to find reliable biomarkers in this manner. For the mentioned ratio of features to subjects there are always some features that appear to be different between the groups. A solution may be sought in multi-feature biomarkers (e.g. the expression pattern of multiple genes), but for larger patterns the number of biomarker such candidates grows rapidly to infinity, and within this wealth of choice it is again very likely to find so-called "spurious" patterns, i.e. patterns that show a clear difference between the patients that were used in the original biomarker discovery studies, but which are not able to classify new, as yet unseen subjects. To make matters worse, the poor measurement accuracy of for example micro-array experiments makes existing "true" differences harder to spot.

Based on this background it was an object of the present invention to provide means for a more reliable detection of biomarkers for different patient groups.

This object is achieved by a method according to claim 1, a computer program according to claim 3, and a record carrier according to claim 4. Preferred embodiments are disclosed in the dependent claims.

The proposed method shall serve for the determination of biomarkers for different patient groups with respect to a given disease. The terms "patient" and "disease" shall be understood in this context in a broad sense, i.e. representing (human or animal) living organisms and arbitrary (healthy or non-healthy) physiological states, respectively. Moreover, it should be noted that the mentioned different patient groups are selected such that they represent different categories of the considered disease, wherein these categories shall be differentiated with the help of the biomarkers that are looked for. The method comprises the following steps:
- The definition of a basic computational model for the disease, wherein said model comprises a number M of interacting nodes. The term "node" shall denote in this context any quantity or state variable that is part of the model, for example the concentration of a specific molecule, the activity of a gene or the like. The basic model may for example be formulated with ordinary differential equations. Most preferably, it is formulated as a Bayesian network.
- The creation of group-specific models by adapting copies of the basic model to data from each of the different patient groups. The basic model may for example comprise a plurality of parameters that are fitted to the data of the different patient groups to yield the group-specific models.
- The identification of "local models" within the specific models, wherein the local models by definition comprise a smaller number n < M of directly interacting nodes. The identification of the local models is typically done by an analysis of the model structure or model definition. As the basic model and the specific models have the same structure, analysis may of course also be done at the basic model and be transferred to the specific models derived from it. The directly interacting nodes typically represent quantities that have a strong correlation, as e.g. in the case of molecular reaction partners.
- The comparison of local models with the same nodes but lying within different group-specific models, and the selection of those of these local models as biomarkers that show the largest differences between the groups. Thus the predictions of corresponding local models within the different group-specific models are for example compared when these group-specific models are fed with the same input data; predictions that show the largest variations between the considered groups are then likely to be good biomarkers.

The method has the advantage that the selection of the biomarkers is based on local models which comprise quantities that are comparatively tightly related to each other. This helps to distinguish truly group-specific effects, which are likely to affect all such quantities of the local models similarly, from only random fluctuations of single quantities.

The invention further relates to a computer program for enabling carrying out a method of the kind described above.

Finally, the invention comprises a record carrier, for example a floppy disk, a hard disk, or a compact disc (CD), on which a computer program of the aforementioned kind is stored.

These and other aspects of the invention will be apparent from and elucidated with reference to the embodiment(s) described hereinafter. These embodiments will be described by way of example with the help of the accompanying drawings in which:
- Figure 1: shows a part of a Bayesian network model for the anti-apoptotic TNFs/NF-kB/IAP pathway, wherein a selection of three connected nodes that can be used to form a marginalized local model (dotted circle);
- Figure 2: shows schematically the layout of a software module implementing the method of the present invention.

A considerable research effort has been made in modeling molecular diagnostics data such as genomics and proteomics measurements through Bayesian and Markov networks (cf. e.g. Jaimovich, A., Elidan, G., Margalit, H. and Friedman, N. (2006): "Towards an integrated protein-protein interaction network: a relational Markov network approach", Journal of Computational Biology 13(2):145-64; Husmeier, D. (2003): "Sensitivity and specificity of inferring genetic regulatory interactions from microarray experiments with dynamic Bayesian networks", Bioinformatics 19(17):2271-82; Tamada, Y., Bannai, H., Imoto, S., Katayama, T., Kanehisa, M. and Miyano, S. (2005): "Utilizing evolutionary information and gene expression data for estimating gene networks with bayesian network models", Journal of Bioinformatics and Computational Biology 3(6):1295-313; Le Phillip, P., Bahl, A. and Ungar, L. (2004): "Using prior knowledge to improve genetic network reconstruction from microarray data", In Silico Biology 4(3):335-53).

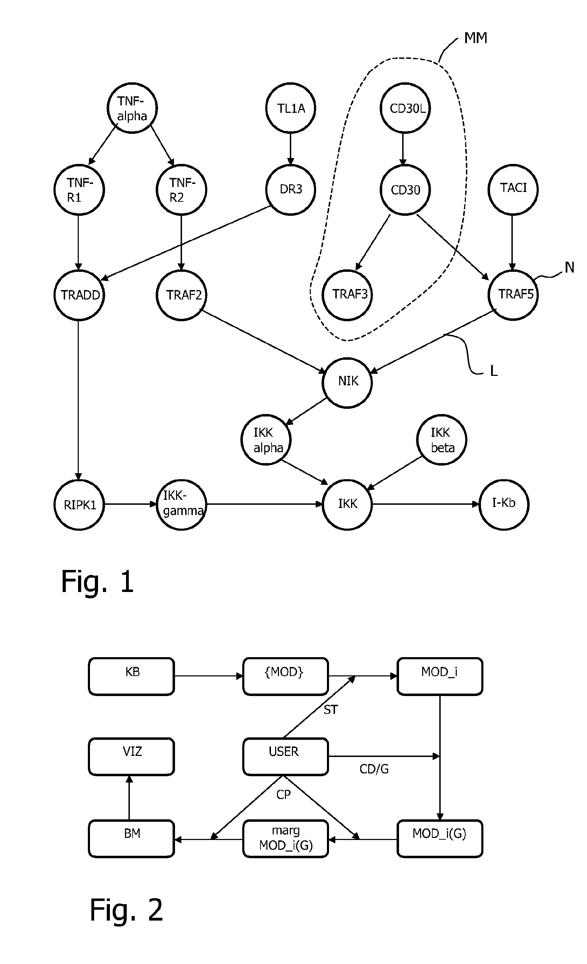

Figure 1 shows in this respect a part of a Bayesian network model for the anti-apoptotic TNFs/NF-kB/IAP pathway, wherein the nodes N represent particular molecules and their concentrations, while the links L between that nodes represent molecular interactions. As will be described in the following, computational models of this kind can be used to identify biomarkers that are relevant for specific patient groups. The proposed method makes use of existing knowledge about the examined paradigm and proceeds along the following lines:
- First, a number of biochemical pathways that are known to be relevant for the underlying disease or condition are selected, along with all the corresponding knowledge from literature, databases, etc.
- This knowledge is transformed into a number of *in silico* (computer) models corresponding to different aspects of the underlying disease. It is proposed that such models are implemented in the form of (dynamic) Bayesian networks.
- Each model is duplicated into two or more models, one for each of the groups that the biomarker (or set of biomarkers) is supposed to identify. Initially, each of the duplicates is identical and is defined purely from existing background knowledge.
- Where possible, the duplicate models are updated with respect to the data that is available for each group of patients (e.g. gene expression or proteomics data), thus creating a group-specific version of each model. This is of course only possible if the variables in the models at least partially overlap with the available measurements. The Bayesian framework is well suited to combine a priori knowledge (the general model) and experimental observations into an updated model. A wide literature already exists on this topic, including (possibly user-defined) ways to balance the importance of old knowledge and new data.
- The resulting models can be subdivided into smaller "marginal" models of locally interacting or interdependent molecules. In a Bayesian network model like that shown in Figure 1, this is relatively straightforward. Interacting/interdependent entities are represented by two circles (nodes N) that are connected by a line (edge L). If, for example, biomarkers of three features are searched for, one may select all groups of three nodes N that are interconnected. If the number of such groups is too high, it is possible to consider only those that are connected most strongly(havethe strongest interaction). Restricting the model to a connected subgroup of nodes ("marginalization") is a standard part of Bayesian network theory.
- After marginalization, local models that contain the same nodes are compared to each other for different subject groups. Those that feature the highest difference (implemented e.g. as the Kullback-Leibler divergence) between the groups are output as the most likely biomarker candidates.

The advantage of this approach is that a pre-selection of biomarker candidates is made, based on the available biological knowledge. Entities that are known to be interdependent are also likely to be affected together under the same condition (e.g. the presence of a specific disease). A difference in measured values between different groups may be to weak to be picked up (due to measurement inaccuracy) for a single entity, but may emerge more strongly when several connected entities are combined. Alternatively, a small difference in a single entity that arrives by chance (which is always possible for large numbers of measured features and low numbers of subjects in which they are measured) will no longer be (erroneously) accepted as a biomarker hypothesis if the difference is not present in any of the entities that it interacts with.

In a more general sense, current practice in biomarker detection can be seen as the construction of a model based purely on measurement data, where the model has a statement about a patient as its output and the full range of clinical patient data as input. This type of model construction is known to be vulnerable to "overfitting" (creating models that perform well on the (limited) data from the clinical study, but perform poorly on new data). Inclusion of a priori knowledge, e.g. in a Bayesian approach, has often been shown strongly to reduce this effect.

Figure 2 shows a possible implementation of the described method as a "Biomarker Suite". This software solution contains a biomedical knowledge base KB (including e.g. pathway databases and curated databases that describe molecular interactions/reactions found in literature), a number of molecular models {MOD} (including pathway models and more specialized in silico disease models) and a search engine that links a selection of models to a search term ST (e.g. a disease). A user (USER) of the Biomarker Suite would insert one (or several) search terms ST related to the paradigm for which biomarkers are sought, with which a suitable model MOD_i is selected. The user would further upload a set of relevant measurements for two or more patient groups (clinical data per subject group, CD/G) through a dedicated "upload wizard", and fill in a form of further control parameters CP, such as the desired size (or size range) of the biomarkers, the minimum/maximum number of biomarkers and the confidence that is required for a biomarker to be proposed. The system then performs the steps that are mentioned above internally, making a transition from group-adapted models MOD_i(G) to marginalized models margMOD_i(G), and finally output a set of multi-feature biomarker candidates BM. The candidates BM are visualized (VIZ) as part of the model that they are part of (as in Figure 1), the observed marginal model difference between the groups is plotted and further, more standard methods such as principal component analysis and clustering can be used to indicate the effect of each proposed biomarker. The Biomarker Suite is intended for example for use in (academic) research hospitals, pharmaceutical industry and general biomedical research.

In summary, the invention discloses a (computer) model that describes molecular biochemical processes in the body that are relevant for a specific disease and can be based on a combination of a priori knowledge (e.g. from literature) and specific clinical data (e.g. from a disease focused clinical studies). The invention aims to find biomarkers that are relevant for a specific disease by creating different models for different patient groups or categories, e.g. relating to "healthy" and "affected" or to different stages of a disease. Each model is based on the same underlying a priori knowledge, but on different pools of group-specific clinical data. Biomarkers are identified through model comparison, with a special interest for local differences (i.e. in a specific, limited part of the model).

Finally it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

## Claims

1. A method for the determination of biomarkers for different patient groups with respect to a disease, comprising the following steps:
- definition of a basic computational model for the disease comprising a number M of interacting nodes (N);
- creating group-specific models by adapting copies of the basic model to data from each of the different patient groups;
- identifying local models (MM) within the group-specific models that comprise a smaller number n < M of directly interacting nodes;
- comparing local models having the same nodes within the group-specific models and selecting those of them as biomarkers that show the largest differences between the groups.

2. The method according to claim 1,
**characterized in that** the basic model is formulated as a Bayesian network.

3. A computer program for enabling carrying out a method according to claim 1.

4. A record carrier on which a computer program according to claim 3 is stored.
